# EUROPEAN PATENT SPECIFICATION

(11) **EP 4 590 261 B1**
(45) Date of publication and mention of the grant of the patent: **30.09.2026**
(21) Application number: 23805785.5
(22) Date of filing: 12.10.2023
(51) Int. Cl.: A61K 8/34, A61K 8/73, A61Q 17/04

(54) **SUN CARE FORMULATION**
SONNENSCHUTZFORMULIERUNG
FORMULATION DE SOINS SOLAIRES

(30) Priority: 18.10.2022 US 202263416953 P
(43) Date of publication of application: 30.07.2025
(73) Proprietor: Rohm and Haas Company, Collegeville, PA 19426 (US); Dow Silicones Corporation, Midland, Michigan 48686 (US)
(72) Inventor: XU, Wenjun, Collegeville, Pennsylvania 19426 (US); CORTES-CLERGET, Margery, Auburn, Michigan 48611 (US); COURTEMANCHE, Marc-Andre, Auburn, Michigan 48611 (US); DIHANG, Helene, 7180 Seneffe (BE)
(74) Representative: Beck Greener LLP
(86) International application number: PCT/US2023/076634
(87) International publication number: WO 2024/086469

(56) References cited:
- WO-A1-2020/118369
- DATABASE GNPD [online] "MINTEL"; 2 July 2022 (2022-07-02), "Two-Phase After-Sun Spray with Aloe", XP093113021, retrieved from https://www.gnpd.com/sinatra/recordpage/9722548/ Database accession no. 9722548
- DATABASE GNPD [online] "MINTEL"; 8 June 2018 (2018-06-08), "Cooling Gel", XP093113023, retrieved from https://www.gnpd.com/sinatra/recordpage/5734613/ Database accession no. 5734613
- DATABASE GNPD [online] MINTEL; 2 July 2022 (2022-07-02), ANONYMOUS: "Two-Phase After-Sun Spray with Aloe", XP093113021, retrieved from https://www.gnpd.com/sinatra/recordpage/9722548/ Database accession no. 9722548
- DATABASE GNPD [online] MINTEL; 8 June 2018 (2018-06-08), ANONYMOUS: "Cooling Gel", XP093113023, retrieved from https://www.gnpd.com/sinatra/recordpage/5734613/ Database accession no. 5734613
- "Standard Analytical Method E-26", vol. E-26, 1977, CORN REFINERS ASSOCIATION, pages: 1 - 3, XP002810737

## Description

The present invention relates to a sun care formulation. In particular, the present invention relates to a sun care formulation including a dermatologically acceptable carrier; a UV radiation absorbing agent; and a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 2 to 20; wherein the functionalized maltodextrin has a degree of substitution of -Si(R¹)₃ groups, DS, of 1.7 to 3; and wherein the functionalized maltodextrin is free of vinylic carbon.

The damaging effects of sunlight on human skin are well documented. Six percent of the solar energy reaching the Earth's surface is ultraviolet (UV) radiation having a wavelength of 290 to 400 nm. This radiation is divided into two components: (i) low energy UVA radiation having a wavelength of 320 to 400 nm and (ii) high energy UVB radiation having a wavelength of 290 to 320 nm. While the UV portion of solar energy is relatively small, it induces nearly 99% of all the side effects from sunlight exposure. High energy UVB radiation, for example, is responsible for producing sunburn, appearance of skin aging and skin cancer. Low energy UVA radiation, for example, is responsible for inducing direct tanning and erythema (abnormal redness) of the skin and contributes to the appearance of skin aging.

By avoiding direct exposure to sunlight, individuals can avoid the serious effects caused by exposure to UV radiation. However, because of the nature of their work, it is challenging for some people to avoid such exposure. In addition, some people voluntarily expose their skin to the sun, e.g., to tan. Therefore, protection against the harmful effects of the sun is important.

Protection from the harmful effects of UV radiation exposure is available in the form of both topically applied formulations containing at least one physical UV blocker, or at least one chemical UV absorber, or combinations thereof. Physical blockers include active ingredients such as, titanium dioxide, zinc oxide and red petrolatum. Chemical absorbers include active ingredients, such as, paraaminobenzoic acid (more commonly known as PABA), which are generally transparent when applied and act by absorbing UV radiation, offering selective protection against certain UV wave bands, depending on the absorption spectrum of the active ingredient in the formulation.

The effectiveness of a given sunscreen formulation is assessed by how well it protects the skin in terms of a Sun Protection Factor (SPF) which is defined as the ratio of the amount of energy required to produce a minimal erythema on sunscreen protected skin to the amount of energy required to produce the same level of erythema on unprotected skin.

The need for compositions for imparting water resistance and aiding retention of active ingredients in sun care compositions is well known. Without them, sun care actives may wash off wear off, be re-emulsified, or otherwise lose their efficacy.

An approach to imparting water resistance and aiding retention of active ingredients in personal care compositions is disclosed in United States Patent No. 9,486,399 to Zeng et al. Zeng et al. disclose a personal care composition comprising: (a) a polymer, wherein the polymer comprises, as polymerized units, based on the weight of the polymer: 20 parts by weight butyl acrylate/40 parts by weight ethylhexyl acrylate/38.5 parts by weight methyl methacrylate/1.5 parts by weight methacrylic acid/0.075 parts by weight allyl methacrylate; and (b) at least one sun care active; wherein the polymer is formed in a single stage.

Notwithstanding, there remains a need for new film forming ingredients that facilitate the need for imparting water resistance and aiding retention of active ingredients in sun care compositions while simultaneously having an increased bio carbon content when compared to conventional film forming ingredients.

The present invention provides a sun care formulation, comprising: a dermatologically acceptable carrier; a UV radiation absorbing agent; and a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 2 to 20; wherein the functionalized maltodextrin has a degree of substitution of -Si(R¹)₃ groups, DS, of 1.7 to 3; and wherein the functionalized maltodextrin is free of vinylic carbon.

The present invention also provides a method of protecting skin from exposure to the sun, comprising: providing a sun care formulation of the present invention, and applying the sun care formulation to skin.

### DETAILED DESCRIPTION

We have surprisingly found a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, of 2 to 20; wherein the functionalized maltodextrin has a degree of substitution of -Si(R¹)₃ groups, DS, of 1.7 to 3; and wherein the functionalized maltodextrin is free of vinylic carbon; wherein the film forming polymer imparts water resistance and aids in the retention of active ingredients in sun care compositions and wherein the film forming polymer has an increased bio carbon content when compared to conventional film forming ingredients.

Unless otherwise indicated, ratios, percentages, parts, and the like are by weight.

The term "dextrose equivalent, DE" as used herein and in the appended claims refers to the degree of starch hydrolysis, specifically, the reducing value of a starch hydrolysate material compared to the reducing value of an equal weight of dextrose, expressed as percent, dry basis, as measured by the Lane and Eynon method described in Standard Analytical Method E-26, Corn Refiners Association, 6th edition, 1977, E-26, pp. 1-3. For example, a maltodextrin with a DE of 10 would have 10% of the reducing power of dextrose which has a DE of 100.

The term "vinylic carbon" as used herein and in the appended claims refers to a carbon that is involved in a double bond with another carbon.

The term "free of vinylic carbon" as used herein and in the appended claims in reference to the functionalized maltodextrin means that the functionalized maltodextrin contains less than the detectable limit of vinylic carbon.

The term "dermatologically acceptable" as used herein and in the appended refers to ingredients typically used in personal care compositions and is intended to underscore that materials that are toxic when present in the amounts typically found in personal care compositions are not contemplated as part of the present invention.

The term "aesthetic characteristics" as used herein and in the appended claims in reference to a sun care formulation refers to visual and tactile sensory properties (e.g., smoothness, tack, lubricity, texture, color, clarity, turbidity, uniformity).

Preferably, the sun care formulation of the present invention is provided in a product form selected from the group consisting of a cream, a non-aqueous solution, an oil, an ointment, a paste, a gel, a lotion, a milk, a foam, a stick and a suspension. More preferably, the sun care formulation of the present invention is provided as a non-aqueous solution. Most preferably, the sun care formulation of the present invention is formulated for application to skin using a mechanical device (e.g., manual pump spray containers, squeeze bottles) or a pressurized aerosol container (e.g., bag-on-nozzle container, pressurized can) to generate a spray.

Preferably, the sun care formulation of the present invention is provided in a product form selected from the group consisting of a cream, a non-aqueous solution, an emulsion, an oil, an ointment, a paste, a gel, a lotion, a milk, a foam, a stick and a suspension. More preferably, the sun care formulation of the present invention is provided as an emulsion.

Preferably, the sun care formulation of the present invention comprises: a dermatologically acceptable carrier (preferably, 10 to 98 wt% (more preferably, 30 to 92 wt%; still more preferably, 35 to 85 wt%; most preferably, 40 to 80), based on weight of the sun care formulation, of the dermatologically acceptable carrier); a UV radiation absorbing agent (preferably, 0.1 to 70 wt% (more preferably, 5 to 65 wt%; still more preferably, 7.5 to 60 wt%; most preferably, 10 to 55 wt%), based on weight of the sun care formulation, of the UV radiation absorbing agent); and a film forming polymer (preferably, 0.1 to 70 wt% (more preferably, 1 to 15 wt%; still more preferably, 1.5 to 10 wt%; most preferably, 2 to 6 wt%), based on weight of the sun care formulation, of the film forming polymer), wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group (preferably, a methyl group, an ethyl group, a propyl group, a butyl group and a pentyl group; more preferably, a methyl group, an ethyl group, a propyl group and a butyl group; still more preferably, a methyl group, an ethyl group and a propyl group; yet more preferably, a methyl group and an ethyl group; most preferably, a methyl group); wherein the maltodextrin base polymer has a dextrose equivalent, DE, of 2 to 20 (preferably, 4 to 20; more preferably, 6 to 19.75; still more preferably, 14 to 19.5; most preferably, 17 to 19); wherein the functionalized maltodextrin has a degree of substitution of -Si(R¹)₃ groups, DS, of 1.7 to 3 (preferably, 1.8 to 3; more preferably, 2.0 to 3; still more preferably, 2.4 to 2.9; yet more preferably, 2.5 to 2.9; most preferably, 2.6 to 2.9); and wherein the functionalized maltodextrin is free of vinylic carbon. More preferably, the sun care formulation of the present invention comprises: a dermatologically acceptable carrier (preferably, 10 to 98 wt% (more preferably, 30 to 92 wt%; still more preferably, 35 to 85 wt%; most preferably, 40 to 80), based on weight of the sun care formulation, of the dermatologically acceptable carrier); a UV radiation absorbing agent (preferably, 0.1 to 70 wt% (more preferably, 5 to 65 wt%; still more preferably, 7.5 to 60 wt%; most preferably, 10 to 55 wt%), based on weight of the sun care formulation, of the UV radiation absorbing agent); and a film forming polymer (preferably, 0.1 to 70 wt% (more preferably, 1 to 15 wt%; still more preferably, 1.5 to 10 wt%; most preferably, 2 to 6 wt%), based on weight of the sun care formulation, of the film forming polymer), wherein the film forming polymer is a functionalized maltodextrin comprising a maltodextrin base polymer functionalized with - Si(R¹)₃ groups; wherein the -Si(R¹)₃ groups are linked to the maltodextrin base polymer through a C-O-Si bond; wherein each R¹ is independently a C₁₋₁₀ linear or branched, saturated alkyl group (preferably, a methyl group, an ethyl group, a propyl group, a butyl group and a pentyl group; more preferably, a methyl group, an ethyl group, a propyl group and a butyl group; still more preferably, a methyl group, an ethyl group and a propyl group; yet more preferably, a methyl group and an ethyl group; most preferably, a methyl group); wherein the maltodextrin base polymer has a dextrose equivalent, DE, of 2 to 20 (preferably, 4 to 20; more preferably, 6 to 19.75; still more preferably, 14 to 19.5; most preferably, 17 to 19); wherein the functionalized maltodextrin has a degree of substitution of -Si(R¹)₃ groups, DS, of 1.7 to 3 (preferably, 1.8 to 3; more preferably, 2.0 to 3; still more preferably, 2.4 to 2.9; yet more preferably, 2.5 to 2.9; most preferably, 2.6 to 2.9); and wherein the functionalized maltodextrin is free of vinylic carbon.

Preferably, the sun care formulation of the present invention, comprises 10 to 98 wt% (preferably, 30 to 92 wt%; more preferably, 35 to 85 wt%; most preferably, 40 to 80), based on weight of the sun care formulation, of a dermatologically acceptable carrier. More preferably, the sun care formulation of the present invention, comprises 10 to 98 wt% (preferably, 30 to 92 wt%; more preferably, 35 to 85 wt%; most preferably, 40 to 80), of a dermatologically acceptable carrier; wherein the dermatologically acceptable carrier is selected from the group consisting of water; glycols (e.g., ethylene glycol, propylene glycol, butylene glycol, pentylene glycol, hexylene glycol, dipropylene glycol, ethoxydiglycol); C₁₋₁₀ straight or branched chain alcohols (e.g., methyl alcohol, ethyl alcohol, propyl alcohol, isopropyl alcohol, butyl alcohol, 2-butoxyethanol); ketones (e.g., acetone); acetates (e.g., methyl acetate); butyl cellusolve; dimethicones; polydimethylsiloxanes; alkanes (e.g., isododecane, isohexane); alkanoates (e.g., methyl undecanoate), dermatologically acceptable hydrophobic ester oils (e.g., caprylic capric triglycerides); dicaprylyl carbonate; alkyl benzoates (e.g., C₁₂₋₁₅ alkyl benzoate); hemisqualane; dioctylether; keto acids (e.g., levulinic acid) and mixtures thereof. Still more preferably, the sun care formulation of the present invention, comprises 10 to 98 wt% (preferably, 30 to 92 wt%; more preferably, 35 to 85 wt%; most preferably, 40 to 80), of a dermatologically acceptable carrier; wherein the dermatologically acceptable carrier is selected to be capable of evaporating upon application of the sun care formulation to the skin (preferably, human skin). Yet more preferably, the sun care formulation of the present invention, comprises 10 to 98 wt% (preferably, 30 to 92 wt%; more preferably, 35 to 85 wt%; most preferably, 40 to 80), of a dermatologically acceptable carrier; wherein the dermatologically acceptable carrier includes a C₁₋₄ straight or branched chain alcohol (e.g., methyl alcohol, ethyl alcohol, propyl alcohol, isopropyl alcohol, butyl alcohol) (preferably, wherein the alcohol is a denatured alcohol). Most preferably, the sun care formulation of the present invention, comprises 10 to 98 wt% (preferably, 30 to 92 wt%; more preferably, 35 to 85 wt%; most preferably, 40 to 80), of a dermatologically acceptable carrier; wherein the dermatologically acceptable carrier includes a specifically denatured ethyl alcohol (e.g., INCI: SD alcohol 40-B; INCI: Alcohol Denat.).

Preferably, the sun care formulation of the present invention, comprises 0.1 to 70 wt% (preferably, 5 to 65 wt%; more preferably, 7.5 to 60 wt%; most preferably, 10 to 55 wt%), based on weight of the sun care formulation, of a UV radiation absorbing agent. More preferably, the sun care formulation of the present invention, comprises 0.1 to 70 wt% (preferably, 5 to 65 wt%; more preferably, 7.5 to 60 wt%; most preferably, 10 to 55 wt%), of a UV radiation absorbing agent; wherein the UV radiation absorbing agent is selected from the group including physical blockers (e.g., red petrolatum, titanium dioxide, zinc oxide); chemical absorbers (e.g., 1-(4-methoxyphenol)-3-(4-tert-butylphenyl)propane-1,3-dione (INCI: avobenzone), 2-hydroxy-4-methoxybenzophenone (INCI: oxybenzone); dioxybenzone; sulisobenzone; menthyl anthranilate; para-aminobenzoic acid; amyl paradimethylaminobenzoic acid; octyl para-dimethylaminobenzoate; ethyl 4-bis (hydroxypropyl) para-aminobenzoate; polyethylene glycol (PEG-25) para-aminobenzoate; ethyl 4-bis (hydroxypropyl) aminobenzoate; diethanolamine para-methyoxycinnamate; 2-ethoxyethyl para-methoxycinnamate; ethylhexyl para-methoxycinnamate; octyl para-methoxycinnamate; isoamyl para-methoxycinnamate; 2-ethylhexyl-2-cyano-3,3-diphenyl-acrylate; 2-ethylhexyl-2-cyano-3,3-diphenyl-2-propenoate (INCI: octocrylene); 2-ethylhexyl 2-hydroxybenzoate (INCI: octisalate); homomenthyl salicylate; glyceryl aminobenzoate; triethanolamine salicylate; digalloyl trioleate; lawsone with dihydroxyacetone; 2-phenylbenzimidazole-5-sulfonic acid; 4-methylbenzylidine camphor; avobenzone; triazines; benzotriazoles; vinyl group-containing amides; cinnamic acid amides; sulfonated benzimidazoles); 3,3,5-trimethylcyclohexyl 2-hydroxybenzoate (INCI: Homosalate)); and mixtures thereof. Still more preferably, the sun care formulation of the present invention, comprises 0.1 to 70 wt% (preferably, 5 to 65 wt%; more preferably, 7.5 to 60 wt%; most preferably, 10 to 55 wt%), of a UV radiation absorbing agent, wherein the UV radiation absorbing agent comprises a mixture of UV radiation absorbing agents. Yet more preferably, the sun care formulation of the present invention, comprises 0.1 to 70 wt% (preferably, 5 to 65 wt%; more preferably, 7.5 to 60 wt%; most preferably, 10 to 55 wt%), of a UV radiation absorbing agent, wherein the UV absorbing agent is a mixture of UV absorbing agents including at least one of 1-(4-methoxyphenol)-3-(4-tert-butylphenyl)propane-1,3-dione (INCI: avobenzone); 2-ethylhexyl 2-hydroxybenzoate (INCI: octisalate); 2-ethyhexyl-2-cyano-3,3-diphenyl-2-propenoate (INCI: octocrylene); 2-hydroxy-4-methoxybenzophenone (INCI: oxybenzone) and 3,3,5-trimethylcyclohexyl 2-hydroxybenzoate (INCI: Homosalate). Most preferably, the sun care formulation of the present invention, comprises 0.1 to 70 wt% (preferably, 5 to 65 wt%; more preferably, 7.5 to 60 wt%; most preferably, 10 to 55 wt%), of a UV radiation absorbing agent, wherein the UV absorbing agent is a mixture of UV absorbing agents including 1-(4-methoxyphenol)-3-(4-tert-butylphenyl)propane-1,3-dione (INCI: avobenzone); 2-ethylhexyl 2-hydroxybenzoate (INCI: octisalate); 2-ethyhexyl-2-cyano-3,3-diphenyl-2-propenoate (INCI: octocrylene) and 3,3,5-trimethylcyclohexyl 2-hydroxybenzoate (INCI: Homosalate).

Preferably, the sun care formulation of the present invention, comprises 0.1 to 70 wt% (preferably, 1 to 15 wt%; more preferably, 1.5 to 10 wt%; most preferably, 2 to 6 wt%), based on weight of the sun care formulation, of the film forming polymer; wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group (preferably, a methyl group, an ethyl group, a propyl group, a butyl group and a pentyl group; more preferably, a methyl group, an ethyl group, a propyl group and a butyl group; still more preferably, a methyl group, an ethyl group and a propyl group; yet more preferably, a methyl group and an ethyl group; most preferably, a methyl group); wherein the maltodextrin base polymer has a dextrose equivalent, DE, 2 to 20 (preferably, 4 to 20; more preferably, 6 to 19.75; still more preferably, 14 to 19.5; most preferably, 17 to 19); wherein the functionalized maltodextrin has a degree of substitution of -Si(R¹)₃ groups, DS, of 1.7 to 3 (preferably, 1.8 to 3; more preferably, 2.0 to 3; still more preferably, 2.4 to 2.9; yet more preferably, 2.5 to 2.9; most preferably, 2.6 to 2.9); and wherein the functionalized maltodextrin is free of vinylic carbon. More preferably, the sun care formulation of the present invention, comprises 0.1 to 70 wt% (preferably, 1 to 15 wt%; more preferably, 1.5 to 10 wt%; most preferably, 2 to 6 wt%), based on weight of the sun care formulation, of a film forming polymer; wherein the film forming polymer is a functionalized maltodextrin comprising a maltodextrin base polymer functionalized with - Si(R¹)₃ groups; wherein the -Si(R¹)₃ groups are linked to the maltodextrin base polymer through a C-O-Si bond; wherein each R¹ is independently a C₁₋₁₀ linear or branched, saturated alkyl group (preferably, a methyl group, an ethyl group, a propyl group, a butyl group and a pentyl group; more preferably, a methyl group, an ethyl group, a propyl group and a butyl group; still more preferably, a methyl group, an ethyl group and a propyl group; yet more preferably, a methyl group and an ethyl group; most preferably, a methyl group); wherein the maltodextrin base polymer has a dextrose equivalent, DE, 2 to 20 (preferably, 4 to 20; more preferably, 6 to 19.75; still more preferably, 14 to 19.5; most preferably, 17 to 19); wherein the functionalized maltodextrin has a degree of substitution of -Si(R¹)₃ groups, DS, of 1.7 to 3 (preferably, 1.8 to 3; more preferably, 2.0 to 3; still more preferably, 2.4 to 2.9; yet more preferably, 2.5 to 2.9; most preferably, 2.6 to 2.9); and wherein the functionalized maltodextrin is free of vinylic carbon.

Preferably, the maltodextrin base polymer has a dextrose equivalent, DE, 2 to 20 (preferably, 4 to 20; more preferably, 6 to 19.75; still more preferably, 14 to 19.5; most preferably, 17 to 19). More preferably, the maltodextrin base polymer has a dextrose equivalent, DE, 2 to 20 (preferably, 4 to 20; more preferably, 6 to 19.75; still more preferably, 14 to 19.5; most preferably, 17 to 19); wherein the maltodextrin base polymer is a straight or branched chain maltodextrin polymer comprising a plurality of glucose structural units. Most preferably, the maltodextrin base polymer has a dextrose equivalent, DE, 2 to 20 (preferably, 4 to 20; more preferably, 6 to 19.75; still more preferably, 14 to 19.5; most preferably, 17 to 19); wherein the maltodextrin base polymer is a straight or branched chain maltodextrin polymer comprising a plurality of glucose structural units; wherein 90 to 100 mol% (preferably, 92 to 100 mol%; more preferably, 93 to 100 mol%; most preferably, 94.5 to 100 mol%) of the glucose structural units are connected by α-1,4 linkages and 0 to 10 mol% (preferably, 0 to 8 mol%; more preferably, 0 to 7 mol%; most preferably, 0 to 5.5 mol%) of the glucose structural units are connected by α-1,6 linkages.

Preferably, the maltodextrin base polymer contains less than 0.01 wt%, based on weight of the maltodextrin base polymer, of alternan. More preferably, the maltodextrin base polymer contains less than 0.001 wt%, based on weight of the maltodextrin base polymer, of alternan. Most preferably, the maltodextrin base polymer contains less than the detectable limit of alternan.

Preferably, < 0.1 mol% (preferably, < 0.01 mol%; more preferably, < 0.001 mol%; most preferably, < detectable limit) of the glucose structural units in the maltodextrin base polymer are connected by β-1,4 linkages.

Preferably, < 0.1 mol% (preferably, < 0.01 mol%; more preferably, < 0.001 mol%; most preferably, < detectable limit) of the glucose structural units in the maltodextrin base polymer are connected by β-1,3 linkages.

Preferably, the sun care formulation of the present invention optionally further comprises 0 to 90 wt% (preferably, 0.01 to 65 wt%; more preferably, 1 to 50 wt%; preferably, 5 to 25 wt%), based on weight of the sun care formulation, of a color ingredient. More preferably, the sun care formulation of the present invention optionally further comprises 0 to 90 wt% (preferably, 0.01 to 65 wt%; more preferably, 1 to 50 wt%; preferably, 5 to 25 wt%), based on weight of the sun care formulation, of a color ingredient; wherein the color ingredient is selected from the group consisting of inorganic pigments, organic pigments, aqueous pigment dispersions and mixtures thereof. Still more preferably, the sun care formulation of the present invention optionally further comprises 0 to 90 wt% (preferably, 0.01 to 65 wt%; more preferably, 1 to 50 wt%; preferably, 5 to 25 wt%), based on weight of the sun care formulation, of a color ingredient; wherein the color ingredient is selected from the group consisting of Ext. D&C Yellow No. 2, Ext. D & C Violet No. 2, FD&C Red No. 4, FD&C Red No. 40, FD&C Yellow No. 5, FD&C Yellow No. 6, FD&C Green No. 3, FD&C Blue No. 1, D&C Yellow No. 7, D&C Yellow No. 8, D&C Yellow No. 10, D&C Yellow No. 11, D&C Violet No. 2, D&C Red No. 6, D&C Red No. 7, D&C Red No. 17, D&C Red No. 21, D&C Red No. 22, D&C Red No. 27, D&C Red No. 28, D&C Red No. 30, D&C Red No. 31, D&C Red No. 34, D&C Red No. 33, D&C Red No. 36, D&C Green No. 5, D&C Green No. 6, D&C Green No. 8, D&C Blue No. 4, D&C Orange No. 4, D&C Orange No. 5, D&C Orange No. 10, D&C Orange No. 11, D&C Brown No. 1, Aluminum powder, Annatto, Bismuth citrate, Bismuth Oxychloride, Bronze powder, Caramel, Carmine, β-Carotene, Chromium hydroxide green, Chromium oxide green, Copper chlorophyllin, Copper powder, Dihydroxyacetone, Ferric Ammonium ferrocyanide, Ferric ferrocyanide, Guanine, Iron oxide, Manganese Violet, Mica, Silver, Titanium Dioxide, Ultramarine, Zinc Oxide and mixtures thereof. Still more preferably, the sun care formulation of the present invention optionally further comprises 0 to 90 wt% (preferably, 0.01 to 65 wt%; more preferably, 1 to 50 wt%; preferably, 5 to 25 wt%), based on weight of the sun care formulation, of a color ingredient; wherein the color ingredient includes at least one iron oxide. Most preferably, the sun care formulation of the present invention optionally further comprises 0 to 90 wt% (preferably, 0.01 to 65 wt%; more preferably, 1 to 50 wt%; preferably, 5 to 25 wt%), based on weight of the sun care formulation, of a color ingredient; wherein the color ingredient includes a mixture of iron oxides.

Preferably, the sun care formulation of the present invention, optionally, further comprises an additive. More preferably, the sun care formulation of the present invention, further comprises an additive, wherein the additive is selected from the group consisting of water proofing agents, emollients, preservatives, antioxidants, fragrances, humectants, rheology modifiers, aesthetic modifiers, vitamins, skin protectants, oils (e.g., a hydrophobic ester oil, such as caprylic/capric triglyceride), emulsifiers, surfactants, pearlizers, consistency factors, thickeners, super fatting agents, stabilizers, polymers, silicone compounds, fats, waxes, lecithins, phospholipids, fillers, light management powders, antiperspirant actives (e.g., aluminum salts, zirconium salts) and mixtures thereof.

Preferably, the sun care formulation of the present invention has a pH of 4 to 9. More preferably, the sun care formulation of the present invention has a pH of 4.5 to 8.5. Still more preferably, the sun care formulation of the present invention has a pH of 5.0 to 8.0. Most preferably, the sun care formulation of the present invention has a pH of 5.5 to 7.5.

Some embodiments of the present invention will now be described in detail in the following **Examples.**

### Synthesis S1: Silylated Maltodextrin

Ammonium chloride (412.4 mg, 0.05 eq) and Glucidex^{®} 1 maltodextrin (DE 1 from Roquette) (25.0 g, 0.154 mol, 1.0 eq) were combined in a 2 CV Helicone mixer (CIT) with a stirring speed at 5 Hz. The resulting mixture was transferred to a reactor. Hexamethyldisilazane (80.87 g, 3.25 eq) was then added to the reactor contents dropwise. Dimethyl sulfoxide (10.8 g) was then added to the reactor contents. The reactor was then sealed and continuously flushed with nitrogen. The reactor contents were stirred at 20 Hz. Heat was applied to the reactor using a heating mantle set to a temperature of 40 °C and the stirring was increased to 50 Hz. After 30 min, the heating mantle was set to 50 °C. The temperature setting for the heating mantle was then increased to 80 °C over the course of 1 h by 10 °C increments. The reactor contents were stirred for 1 hr after the temperature of the reactor contents reached 71 °C. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~46.3 g). The degree of substitution, DS, of the -Si(CH₃)₃ on the maltodextrin base polymer was determined by ¹H NMR to be 1.67.

### Synthesis S2: Silylated Maltodextrin

Ammonium chloride (454.4 mg, 0.05 eq) and Glucidex^{®} 1 maltodextrin (DE 1 from Roquette) (27.0 g, 0.166 mol, 1.0 eq) were combined in a 2 CV Helicone mixer (CIT) with a stirring speed at 5 Hz. The resulting mixture was transferred to a reactor. Hexamethyldisilazane (87.34 g, 3.25 eq) was then added to the reactor contents dropwise. Dimethyl sulfoxide (11.66 g) was then added to the reactor contents. The reactor was then sealed and continuously flushed with nitrogen. The reactor contents were stirred at 20 Hz. Heat was applied to the reactor using a heating mantle set to a temperature of 92 °C and the stirring was increased to 50 Hz. The reactor contents were stirred for 1.5 hr after the temperature of the reactor contents reached 83 °C. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~56.6 g). The degree of substitution, DS, of the -Si(CH₃)₃ on the maltodextrin base polymer was determined by ¹H NMR to be 2.1.

### Synthesis S3: Silylated Maltodextrin

Ammonium chloride (445.4 mg, 0.05 eq) and Glucidex^{®} 1 maltodextrin (DE 1 from Roquette) (27.0 g, 0.167 mol, 1.0 eq) were combined in a 2 CV Helicone mixer (CIT) with a stirring speed at 5 Hz. The resulting mixture was transferred to a reactor. Hexamethyldisilazane (60.47 g, 2.25 eq) was then added to the reactor contents dropwise. Dimethyl sulfoxide (11.7 g) was then added to the reactor contents. The reactor was then sealed and continuously flushed with nitrogen. The reactor contents were stirred at 20 Hz. Heat was applied to the reactor using a heating mantle set to a temperature of 55 °C and the stirring was increased to 50 Hz. After 5 min, the heating mantle was set to 102 °C. The reactor contents were stirred for 2 hr. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~407.3 g). The degree of substitution, DS, of the -Si(CH₃)₃ on the maltodextrin base polymer was determined by ¹H NMR to be 2.55.

### Synthesis S4: Silylated Maltodextrin

To a 25 mL scintillation vial was added ammonium chloride (33.0 mg, 0.05 eq) and Maltrin M250 maltodextrin (DE 23-27 from Grain Processing Corporation) (2.0 g, 12.3 mM, 1.0 eq). Hexamethyldisilazane (4.48 g, 2.25 eq) was then added dropwise to the vial contents. Dimethyl sulfoxide (1 g) was then added to the vial contents, and the vial was capped with a screw cap septum topped with two vent needles. The vial was placed on an aluminum heating block set at 85 °C for 1.5 hrs. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~4.15 g). The degree of substitution, DS, of the -Si(CH₃)₃ on the maltodextrin base polymer was determined by ¹H NMR to be 2.2.

### Synthesis S5: Silylated Cellulose

Polysaccharide (BioFloc XV, 15.0 g, from Tartas) was weighed in a 2 L three-neck flask equipped with a nitrogen inlet and a temperature controller. Solvent (N,N dimethylacetamide, 331 g, from Sigma-Aldrich) was added and the reaction mixture was placed under an atmosphere of nitrogen with an outlet to avoid over-pressurization of the reactor. Silane (hexamethyldisilazane, 30 g, from The Dow Chemical Company) was added at once to the reaction mixture. The mixture was slowly heated to a set temperature of 130 °C and stirred for 7.5 h. The solution was cooled naturally, then xylenes (600 g, from Sigma-Aldrich) was added to the reaction mixture along with additional hexamethyldisilazane (20 g) and the mixture was stirred for 4 h with a set temperature of 125 °C. The reactor contents were left to cool down to room temperature overnight. The reactor contents were then transferred to a separatory funnel and subjected to non-solvent precipitation by dropwise addition into 2 L of vigorously stirring methanol. The product was isolated by filtration, and dried in a vacuum oven at 50 °C overnight. The product was then suspended in 500 ml of methanol, then filtered and dried in a vacuum oven at 50 °C overnight. The product was analyzed by attenuated total reflection infrared to determine DS at 2.23.

### Synthesis S6: Silylated Cellulose

Polysaccharide (E-60, 15.2 g, from GP Cellulose) was weighed in a 2 L three-neck flask equipped with a nitrogen inlet and a temperature controller. Solvent (N,N dimethylacetamide, 324 g) was added and the reaction mixture was placed under an atmosphere of nitrogen with an outlet to avoid over-pressurization of the reactor. Silane (hexamethyldisilazane, 50.2 g, from The Dow Chemical Company) was added at once to the reaction mixture, along with the saccharin catalyst (850 mg, from Sigma-Aldrich). The mixture was slowly heated to a set temperature of 130°C and stirred for 5 h. The solution was cooled naturally, then xylenes (400 g) were added to the reaction mixture and the mixture was stirred at 120 °C for 8 hours. The reactor contents were left to cool down to room temperature overnight. The cooled reactor contents were then transferred to a separatory funnel and subjected to non-solvent precipitation by dropwise addition into 2 L of vigorously stirring methanol. The product was isolated by filtration and dried in a vacuum oven at 50 °C overnight. The product was then suspended in 500 ml of methanol, then filtered again, and dried in a vacuum oven at 50 °C overnight. was analyzed by attenuated total reflection infrared to determine DS at 2.6.

### Synthesis S7: Silylated Maltodextrin

To a 25 mL scintillation vial was added ammonium chloride (33.0 mg, 0.05 eq) and Maltrin M200 maltodextrin (DE range 16.5-19.9 from Grain Processing Corporation)(2.0 g, 12.3 mM, 1.0 eq). Hexamethyldisilazane (3.58 g, 1.80 eq) was then added dropwise to the vial contents. Dimethyl sulfoxide (0.75 g) was then added to the vial contents and the vial was outfitted with a septum cap with two vent needles. The vial was placed on a heating block set at 80 °C for 1 hr. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~3.6 g). The degree of substitution, DS, of the -Si(CH₃)₃ on the maltodextrin base polymer was determined by ¹H NMR to be 2.76.

### Synthesis S8: Silylated Maltodextrin

To a 25 mL scintillation vial was added ammonium chloride (33.0 mg, 0.05 eq) and Maltrin M040 (DE 4-7 from Grain Processing Corporation) (2.0 g, 12.3 mM, 1.0 eq). Hexamethyldisilazane (4.48 g, 2.25 eq) was then added dropwise to the vial contents. Dimethyl sulfoxide (1 g) was then added to the vial contents and the vial was outfitted with a septum cap with two vent needles. The vial was placed on a heating block set at 85 °C for 1.5 hrs. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~4.15 g). The degree of substitution, DS, of the -Si(CH₃)₃ on the maltodextrin base polymer was determined by ¹H NMR to be 2.5.

### Synthesis S9: Silylated Maltodextrin

Ammonium chloride (445.4 mg, 0.05 eq) and Glucidex^{®} 1 maltodextrin (DE 1 from Roquette) (27.0 g, 0.166 mol, 1.0 eq) were combined in a 2 CV Helicone mixer (CIT) with a stirring speed at 5 Hz. The resulting mixture was transferred to a reactor. Hexamethyldisilazane (87.34 g, 3.25 eq) was then added to the reactor contents dropwise. Dimethyl sulfoxide (11.66 g) was then added to the reactor contents. The reactor was then sealed and continuously flushed with nitrogen. The reactor contents were stirred at 20 Hz. Heat was applied to the reactor using a heating mantle set to a temperature of 50 °C and the stirring was increased to 50 Hz. After 20 min, the heating mantle was set to 100 °C. The reactor contents were stirred for 2 hrs. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~53 g). The degree of substitution, DS, of the -Si(CH₃)₃ on the maltodextrin base polymer was determined by ¹H NMR to be 2.23.

### Synthesis S10: Silylated Maltodextrin

To a 25 mL scintillation vial was added ammonium chloride (33.0 mg, 0.05 eq) and Glucidex^{®} 1 maltodextrin (DE 1 from Roquette) (2.0 g, 12.3 mM, 1.0 eq). Hexamethyldisilazane (4.48 g, 2.25 eq) was then added dropwise to the vial contents. Dimethyl sulfoxide (1 g) was then added to the vial contents and the vial was outfitted with a septum cap with two vent needles. The vial was placed on a heating block set at 85 °C for 2 hrs. The vial contents were then cooled down to < 50 °C and diluted with ethyl acetate (150 mL). The organic layer was transferred to a separatory funnel and washed with distilled water three times (3 x 50 mL). The organic layer was collected in an Erlenmeyer flask, and dried with sodium sulfate. The organic layer was then concentrated under vacuum to yield a fine white powder (~3.96 g). The degree of substitution, DS, of the -Si(CH₃)₃ on the maltodextrin base polymer was determined by ¹H NMR to be 2.51.

### Synthesis S11: Silylated Maltodextrin

Ammonium chloride (445.4 mg, 0.05 eq) and Glucidex^{®} 1 maltodextrin (DE 1 from Roquette) (27.0 g, 0.166 mol, 1.0 eq) were combined in a 2 CV Helicone mixer (CIT) with a stirring speed at 5 Hz. The resulting mixture was transferred to a reactor. Hexamethyldisilazane (87.34 g, 3.25 eq) was then added to the reactor contents dropwise. Dimethyl sulfoxide (11.66 g) was then added to the reactor contents. The reactor was then sealed and continuously flushed with nitrogen. The reactor contents were stirred at 20 Hz. Heat was applied to the reactor using a heating mantle set to a temperature of 50 °C and the stirring was increased to 50 Hz. After 20 min, the heating mantle was set to 100 °C. The reactor contents were stirred for 2 hrs. The heating mantle was then removed and the stirring was decreased to 25 Hz. When the reactor contents cooled to < 50 °C, stirring was stopped and 400 mL of ethyl acetate was added to the reactor contents. Stirring was then resumed at 25 Hz for 5 min. Stirring was then stopped and the organic layer was transferred to a collection jar. Ethyl acetate (100 mL) was then added to the reactor contents and stirring was resumed at 25 Hz for 5 min. Stirring was then stopped and the organic layer was transferred to the contents of the collection jar. The contents of the collection jar was transferred to a separatory funnel and washed with distilled water two times (2 x 250 mL). The organic layer was collected in an Erlenmeyer flask, and dried with sodium sulfate. The organic layer was then concentrated under vacuum to yield a fine white powder (~56.3 g). The degree of substitution, DS, of the -Si(CH₃)₃ on the maltodextrin base polymer was determined by ¹H NMR to be 2.42.

### Synthesis S12: Silylated Maltodextrin

To a 25 mL scintillation vial was added ammonium chloride (24.7 mg, 0.05 eq) and dried Glucidex^{®} 1 maltodextrin (DE 1 from Roquette) (1.5 g, 3.25 eq). Hexamethyldisilazane (4.85 g, 3.25 eq) was then added dropwise to the vial contents. Dimethyl sulfoxide (0.8 g) was then added to the vial contents and the vial was outfitted with a septum cap with two vent needles. The vial was placed on a heating block set at 90 °C for 4 hrs. The vial contents were then cooled down to < 50 °C and diluted with ethyl acetate (200 mL). The organic layer was transferred to a separatory funnel and washed with a 50/50 vol/vol mixture of brine and distilled water three times (3 x 60 mL). The organic layer was collected in an Erlenmeyer flask, and dried with sodium sulfate. The organic layer was then concentrated under vacuum to yield an off white crystalline solid, that was easily reduced to a fine powder with a spatula. The product powder was dried under vacuum in an oven at 50 °C for 5 hrs. The degree of substitution, DS, of the -Si(CH₃)₃ on the maltodextrin base polymer was determined by ¹H NMR to be 2.42.

### Synthesis S13: Silylated Maltodextrin

To a 25 mL scintillation vial was added ammonium chloride (24.7 mg, 0.05 eq) and dried Maltrin M150 maltodextrin (DE 13-17 from Grain Processing Corporation)(1.5 g, 1.0 eq). Hexamethyldisilazane (4.85 g, 3.25 eq) was then added dropwise to the vial contents. Dimethyl sulfoxide (0.8 g) was then added to the vial contents and the vial was outfitted with a septum cap with two vent needles. The vial was placed on a heating block set at 90 °C for 2.5 hrs. The vial contents were then cooled down to < 50 °C and diluted with ethyl acetate (200 mL). The organic layer was transferred to a separatory funnel and washed with a 50/50 vol/vol mixture of brine and distilled water three times (3 x 60 mL). The organic layer was collected in an Erlenmeyer flask, and dried with sodium sulfate. The organic layer was then concentrated under vacuum to yield an off white crystalline solid, that was easily reduced to a fine powder with a spatula. The product powder was dried under vacuum in an oven at 50 °C for 5 hrs. The degree of substitution, DS, of the -Si(CH₃)₃ on the maltodextrin base polymer was determined by ¹H NMR to be 2.64.

### Synthesis S14: Silylated Maltodextrin

To a 25 mL scintillation vial was added ammonium chloride (33.0 mg, 0.05 eq) and Maltrin M200 maltodextrin (DE 20-23 from Grain Processing Corporation)(2.0 g, 1.0 eq). Hexamethyldisilazane (3.58 g, 1.80 eq) was then added dropwise to the vial contents. Dimethyl sulfoxide (0.75 g) was then added to the vial contents and the vial was outfitted with a septum cap with two vent needles. The vial was placed on a heating block set at 80 °C for 1 hrs. The vial contents were then cooled down to < 50 °C and diluted with ethyl acetate (150 mL). The organic layer was transferred to a separatory funnel and washed with a 50/50 vol/vol mixture of brine and distilled water three times (3 x 50 mL). The organic layer was collected in an Erlenmeyer flask, and dried with sodium sulfate. The organic layer was then concentrated under vacuum to yield a fine white powder. The product powder was dried under vacuum in an oven at 50 °C for 5 hrs. The degree of substitution, DS, of the -Si(CH₃)₃ on the maltodextrin base polymer was determined by ¹H NMR to be 2.45.

### Solubility screening (2 wt%)

The solubility of the products of **Syntheses S1-S6** and a commercial maltodextrin with a DE of 1 (Glucidex^{®} 1 from Roquette) were assessed in different carriers by combining individually in separate vials the products of **Syntheses S1-S6** (0.1 g) and the commercial maltodextrin with various solvents (4.9 g) as noted in **TABLE 1.** The resulting 2 wt% solutions were stirred with a magnetic stir bar for 1 hour at ~22 °C. The carriers and solubility observations are provided in **TABLE 1.**

**TABLE 1**

| **Carrier** | **Observation** | | | | | | |
|---|---|---|---|---|---|---|---|
| | **Solute (2 wt%) in carrier** | | | | | | |
| | **S1** | **S2** | **S3** | **S4** | **S5** | **S6** | **Malt⁹** |
| Water | I | I | I | I | I | I | S |
| Ethanol | I | S | S | S | I | I | I |
| Dimethicone, 2 cSt¹ | -- | S | S | S | I | S* | I |
| Isododecane | I | S | S | S | I | I | I |
| Isohexadecane | -- | S | S | -- | -- | -- | -- |
| Methyl undecanoate | -- | S | S | S | -- | -- | -- |
| Triglycerides² | -- | S | S | S | -- | -- | -- |
| Dicaprylyl carbonate³ | -- | S | S | S | -- | -- | -- |
| C₁₂₋₁₅ alkyl benzoate⁴ | -- | S | S | S | I | I | I |
| C₁₃₋₁₅ Alkane⁵ | -- | S | S | S | -- | -- | -- |
| Dioctylether | -- | S | S | S | -- | -- | -- |
| Ethyl PG-Acetal Levulinate⁶ | -- | S | S | -- | -- | -- | -- |
| Homosalates⁷ | -- | S | S | S | -- | -- | -- |
| Sunflower oil⁸ | -- | I | I | S* | I | I | I |
| ¹ DOWSIL^{™} 200 fluid (2 cSt) from The Dow Chemical Company | | | | | | | |
| ² CRODAMOL^{™} GTCC from Croda | | | | | | | |
| ³ CETIOL^{®} CC from BASF | | | | | | | |
| ⁴ CRODAMOL^{™} ABLQ from Croda | | | | | | | |
| ⁵ NEOSSANCE^{®} hemisqualane from Centerchem | | | | | | | |
| ⁶ ECOSMOOTH^{™} universal fluid 1100 from The Dow Chemical Company | | | | | | | |
| ⁷ PARSOL^{®} HMS from DSM | | | | | | | |
| ⁸ Sunflower oil, USDA Certified Organic from MakingCosmetics | | | | | | | |
| ⁹ Glucidex^{®} 1 maltodextran with DE 1 from Roquette. | | | | | | | |
| ^{S} Soluble and clear | | | | | | | |
| ^{S*}Soluble and slightly hazy | | | | | | | |
| ^{I} Insoluble | | | | | | | |

### Solubility screening (50 wt%)

The solubility of the products of **Syntheses S2-S3** and **S7** (2 g) were assessed in isododecane (2 g) as noted in **TABLE 2.** The resulting 50 wt% solutions were stirred with a magnetic stir bar for 1 hour at ~22 °C. The carriers and solubility observations are provided in **TABLE 2.**

**TABLE 2**

| **Carrier** | **Observation** | | |
|---|---|---|---|
| | **Solute (50 wt%) in carrier** | | |
| | **S2** | **S3** | **S7** |
| Isododecane | S | S | S |
| Ethanol | not tested | S | S |
| ^{S} Soluble and clear | | | |

### Viscosity in isododecane

The product of **Synthesis S5-S6** and **S9** was dissolved in isododecane at different concentrations in as noted in **TABLE 3.** The viscosity of the resulting solutions were then determined using a Brookfield DV-111-ultra viscometer, equipped with a SC4-28 spindle at ~ 22 °C and 100 rpm. The results are provided in **TABLE 3.**

**TABLE 3**

| **Solute** | **Concentration (wt%)** | **Viscosity (cP)** |
|---|---|---|
| **Synthesis S5** | 2 | 3,500 |
| **Synthesis S6** | 2 | 3,200 |
| **Synthesis S6** | 5 | gel |
| **Synthesis S9** | 2 | 0 |
| **Synthesis S9** | 5 | 2 |
| **Synthesis S9** | 20 | 5 |
| **Synthesis S9** | 40 | 305 |
| **Synthesis S9** | 50 | 1,260 |

### Comparative Examples CF1-CF6 and Examples F1: Water Repellency

Water repellency of a film is strongly influenced by surface energy. High water repellency is desirable for sun care applications. The water repellency of a formulation can be evaluated by measuring the water contact angle from a deposited film of the formulation. Specifically, films were coated onto a glass slide (50 µm wet thickness) from dispersions formed using the ingredients noted in **TABLE 4** using a doctor blade film applicator with the gap set at 6 mils (0.1524 mm) from the as received polymer solutions, and films were air dried in an environmental controlled room (~22 °C and 50% RH) for at least 72 hours. The water contact angles for the deposited films were then measured (in degrees) at 120 seconds after water droplets were deposited on the substrate using a drop shape analyzer (Kruss DSA100). The results of the water contact angle measurements are provided in **TABLE 4.** Higher contact angles indicate greater water repellency. Contact angles of above 90° are considered excellent.

**TABLE 4**

| **Example** | **Ingredients** | | | | | **Water Contact angle** |
|---|---|---|---|---|---|---|
| | **Polymer** | | | | **Isododecane** | |
| | **Type** | **DE** | **DS** | **(wt%)** | **(wt%)** | |
| **Comp. Ex. CF1** | Silicone Acrylate¹ | -- | -- | 2 | 98 | 102.3 |
| **Comp. Ex. CF2** | Modified pullulan² | -- | -- | 2 | 98 | 90.7 |
| **Comp. Ex. CF3** | Ethyl cellulose³ | -- | -- | 2 | 98 | 66.3 |
| **Comp. Ex. CF4** | **Synthesis S11** | 1 | 2.42 | 2 | 98 | 94.2 |
| **Comp. Ex. CF5** | **Synthesis S12** | 1 | 2.42 | 2 | 98 | 93.8 |
| **Comp. Ex. CF6** | **Synthesis S3** | 1 | 2.55 | 2 | 98 | 114.4 |
| **Example F1** | **Synthesis S13** | 13-17 | 2.64 | 2 | 98 | 101.5 |
| ¹ DOWSIL^{™} FA4012 ID acrylates/polytrimethylsiloxy-methacrylate copolymer from The Dow Chemical Company | | | | | | |
| ² TSPL-30-ID isododecane (and) trimethylsiloxysilylcarbamoyl pullan from Shin-Etsu Chemical Co., Ltd. | | | | | | |
| ³ ETHOCEL^{™} 7 from DuPont | | | | | | |

### Comparative Examples CF7-CF12 and Examples F2-F4: Sun care formulations

Sun care formulations were prepared having compositions as noted in **TABLE 5.** The avobenzone, caprylic/capric triglyceride and ethylhexyl salicylate were mixed in a flask and heated to 60 °C until all the avobenzone was melted. The heat source was removed and the rest of the ingredients other than the ethanol were added to the contents of the flask. When the flask contents cooled to < 30 °C, the ethanol was added with stirring for 30 minutes until the flask contents were homogeneous.

**TABLE 5**

| **Ingredient** | **CF7** | **CF8** | **CF9** | **CF10** | **CF11** | **CF12** | **CF13** | **F2** | **F3** | **F4** |
|---|---|---|---|---|---|---|---|---|---|---|
| | **wt%** | | | | | | | | | |
| Ethanol | q.s. 100 | | | | | | | | | |
| Caprylic/Capric Triglyceride¹ | 5 | 5 | 5 | 5 | 5 | 5 | 5 | 5 | 5 | 5 |
| Homosalate² | 5 | 5 | 5 | 5 | 5 | 5 | 5 | 5 | 5 | 5 |
| Ethylhexyl salicylate³ | 5 | 5 | 5 | 5 | 5 | 5 | 5 | 5 | 5 | 5 |
| Avobenzone⁴ | 3 | 3 | 3 | 3 | 3 | 3 | 3 | 3 | 3 | 3 |
| Octocrylene⁵ | 4 | 4 | 4 | 4 | 4 | 4 | 4 | 4 | 4 | 4 |
| Ethyl cellulose⁶ | -- | 4 | -- | -- | -- | -- | -- | -- | -- | -- |
| Prod. **Syn. S1** | -- | -- | -- | 4 | -- | -- | -- | -- | -- | -- |
| Prod. **Syn. S3** | -- | -- | -- | -- | -- | 4 | -- | -- | -- | -- |
| Prod. **Syn. S6** | -- | -- | 4 | -- | -- | -- | -- | -- | -- | -- |
| Prod. **Syn. S9** | -- | -- | -- | -- | 4 | -- | -- | -- | -- | -- |
| Prod. **Syn. S14** | -- | -- | -- | -- | -- | -- | 4 | -- | -- | -- |
| Prod. **Syn. S13** | -- | -- | -- | -- | -- | -- | -- | -- | 4 | -- |
| Prod. **Syn. S8** | -- | -- | -- | -- | -- | -- | -- | 4 | -- | -- |
| Prod. **Syn. S7** | -- | -- | -- | -- | -- | -- | -- | -- | -- | 4 |
| ¹ From Rita under tradename Ritamollient CCT | | | | | | | | | | |
| ² From DSM under tradename PARSOL^{®} HMS | | | | | | | | | | |
| ³ From DSM under tradename PARSOL^{®} EHS | | | | | | | | | | |
| ⁴ From DSM under tradename PARSOL^{®} 1789 | | | | | | | | | | |
| ⁵ From DSM under tradename PARSOL^{®} 340 | | | | | | | | | | |
| ⁶ From DuPont under tradename ETHOCEL^{™} Standard 7 Premium | | | | | | | | | | |

### In-vitro SPF Performance and Water Resistance

The sun care formulations prepared according to **Comparative Examples CF7-CF8, CF10-CF13** and **Examples F2-F4** were evaluated for SPF performance. The SPF performance of each of the sun care formulations was then tested in triplicate using an *in-vitro* technique according to the following protocol.

The substrate used for the *in-vitro* SPF measurements was a rough PMMA substrate (6 µm - Helioplate HD6 from HelioScreen Co.). The sun care formulations to be tested were each applied to three separate rough 5 cm x 5 cm PMMA substrates at 1.5 mg/cm² using a specific finger spreading method. Each deposited layer of sun care formulation was allowed to dry for at least 60 minutes under ambient conditions in the laboratory. The UV absorption of each dried layer of sun care formulation between 290 nm and 400 nm was then measured at nine (9) separate points using a Labsphere UV-2000S Spectrometer. The initial *in-vitro* SPF, *SPF₁,* value for each sun care formulation prepared according to **Comparative Examples CF7-CF8, CF10-CF13** and **Examples F2-F4** was then calculated based on the results of the UV absorption measurements. The average from the triplicate samples of each sun care formulation prepared according to **Comparative Examples CF7-CF8, CF10-CF13** and **Examples F2-F4** is reported in in **TABLE 6.**

Following the initial SPF testing, the treated PMMA plates were then submerged into a 29 °C, 5 L hard water bath (150 ppm, 2:1 CaCl₂/MgCl₂) with agitation at 600 rpm, using a 1.5 inch oblong smooth magnetic stir bar. The treated PMMA plates were hung, submerged in the water for 15 minutes. The treated PMMA plates were laid flat onto a Kimwipe^{™} wipe with the treated side up and left to dry for 12 hours. After drying each treated PMMA plate was again tested in triplicate to measure the SPF value post immersion, *SPF_{A}.* The average from the triplicate samples for each sun care formulation is reported in **TABLE 6.**

The % SPF retention after the water immersion exposure was then calculated using the following equation ${SPF}_{R} = \frac{{SPF}_{I} - {SPF}_{A}}{{SPF}_{I}} \times 100 %$ wherein *SPF_{R}* is the SPF retention (in %); wherein *SPF_{I}* is the initial SPF measured for the film before immersion in water and wherein *SPF_{A}* is the SPF measured for the film after immersion in water. The results are provided in **TABLE 6.**

**TABLE 6**

| **Sun care Formulation** | **DE** | **DS** | ***SPF_{I}*** | ***SPF_{A}*** | ***SPF_{R}*** |
|---|---|---|---|---|---|
| **Comp. Ex. CF7** | -- | -- | 18.8 | 9.9 | 52.7 |
| **Comp. Ex. CF8** | -- | -- | 25.6 | 21.3 | 83.3 |
| **Comp. Ex. CF10^{a}** | 1 | 1.68 | 21.8 | 10.1 | 46.4 |
| **Comp. Ex. CF11** | 1 | 2.23 | 36.6 | 26.9 | 73.1 |
| **Comp. Ex. CF12** | 1 | 2.55 | 25.4 | 16.9 | 66.6 |
| **Comp. Ex. CF13** | 20-23 | 2.45 | 21.5 | 14.7 | 68.4 |
| **Example F2** | 4-7 | 2.48 | 28.8 | 21.8 | 75.8 |
| **Example F3** | 13-17 | 2.69 | 23.9 | 19.3 | 80.7 |
| **Example F4** | 16.9-19.9 | 2.76 | 15.2 | 15.0 | 98.8 |
| ^{a} Formulation hazy with visible sediments | | | | | |

## Claims

1. A sun care formulation, comprising:
a dermatologically acceptable carrier;
a UV radiation absorbing agent; and
a film forming polymer, wherein the film forming polymer is a functionalized maltodextrin comprising a maltodextrin base polymer functionalized with -Si(R¹)₃ groups; wherein each R¹ is independently a C₁₋₁₀ linear or branched, saturated alkyl group; wherein the maltodextrin base polymer has a dextrose equivalent, DE, as measured by the Lane and Eynon method described in Standard Analytical Method E-26, Corn Refiners Association, 6^{th} edition, 1977, E-26, pp. 1-3, of 2 to 20; wherein the functionalized maltodextrin has a degree of substitution of -Si(R¹)₃ groups, DS, of 1.7 to 3; and wherein the functionalized maltodextrin is free of vinylic carbon.

2. The sun care formulation of claim 1, wherein the dermatologically acceptable carrier is selected from the group consisting of water; glycols; C₁₋₁₀ straight or branched chain alcohols; ketones; acetates; butyl cellusolve; dimethicones; polydimethylsiloxanes; alkanes; alkanoates, dermatologically acceptable hydrophobic ester oils; dicaprylyl carbonate; alkyl benzoates; hemisqualane; dioctylether; keto acids and mixtures thereof.

3. The sun care formulation according to claim 2, wherein the UV radiation absorbing agent is selected from the group consisting of physical blockers, chemical absorbers and mixtures thereof.

4. The sun care formulation according to claim 2, wherein the UV radiation absorbing agent is selected from the group consisting of red petrolatum; titanium dioxide; zinc oxide; 1-(4-methoxyphenol)-3-(4-tert-butylphenyl)propane-1,3-dione; 2-hydroxy-4-methoxybenzophenone; dioxybenzone; sulisobenzone; menthyl anthranilate; para-aminobenzoic acid; amyl paradimethylaminobenzoic acid; octyl para-dimethylaminobenzoate; ethyl 4-bis (hydroxypropyl) para-aminobenzoate; polyethylene glycol (PEG-25) para-aminobenzoate; ethyl 4-bis (hydroxypropyl) aminobenzoate; diethanolamine para-methyoxycinnamate; 2-ethoxyethyl para-methoxycinnamate; ethylhexyl para-methoxycinnamate; octyl para-methoxycinnamate; isoamyl para-methoxycinnamate; 2-ethylhexyl-2-cyano-3,3-diphenyl-acrylate; 2-ethylhexyl-2-cyano-3,3-diphenyl-2-propenoate; 2-ethylhexyl-2-hydroxybenzoate; homomenthyl salicylate; glyceryl aminobenzoate; triethanolamine salicylate; digalloyl trioleate; lawsone with dihydroxyacetone; 2-phenylbenzimidazole-5-sulfonic acid; 4-methylbenzylidine camphor; avobenzone; triazines; benzotriazoles; vinyl group-containing amides; cinnamic acid amides; sulfonated benzimidazoles); 3,3,5-trimethylcyclohexyl 2-hydroxybenzoate and mixtures thereof.

5. The sun care formulation according to claim 4, wherein the UV radiation absorbing agent is a mixture of UV absorbing agents including 1-(4-methoxyphenol)-3-(4-tert-butylphenyl)propane-1,3-dione; 2-ethylhexyl 2-hydroxybenzoate; 2-ethyhexyl-2-cyano-3,3-diphenyl-2-propenoate and 3,3,5-trimethylcyclohexyl 2-hydroxybenzoate.

6. The sun care formulation according to claim 5, wherein the dermatologically acceptable carrier includes a C₁₋₄ straight or branched chain alcohol.

7. The sun care formulation according to claim 6, wherein the dermatologically acceptable carrier includes ethanol.

8. The sun care formulation of claim 7, further comprising a dermatologically acceptable hydrophobic ester oil.

9. The sun care formulation of claim 8, further comprising a color ingredient.

## Patentansprüche

1. Sonnenschutzformulierung, umfassend:
einen dermatologisch verträglichen Träger;
ein UV-Strahlung absorbierendes Mittel; und
ein filmbildendes Polymer, wobei das filmbildende Polymer ein funktionalisiertes Maltodextrin ist, das ein mit -Si(R¹)₃-Gruppen funktionalisiertes Maltodextrin-Basispolymer umfasst; wobei jedes R¹ unabhängig eine lineare oder verzweigte gesättigte C₁₋₁₀-Alkylgruppe ist; wobei das Maltodextrin-Basispolymer ein Dextroseäquivalent, DE, von 2 bis 20 aufweist, gemessen nach dem Lane-Eynon-Verfahren, beschrieben in Standard Analytical Method E-26, Corn Refiners Association, 6. Auflage, 1977, E-26, S. 1-3; wobei das funktionalisierte Maltodextrin einen Substitutionsgrad der -Si(R¹)₃-Gruppen, DS, von 1,7 bis 3 aufweist; und wobei das funktionalisierte Maltodextrin frei von vinylischem Kohlenstoff ist.

2. Sonnenschutzformulierung nach Anspruch 1, wobei der dermatologisch verträgliche Träger ausgewählt ist aus der Gruppe bestehend aus Wasser; Glycolen; gerad- oder verzweigtkettigen C₁₋₁₀-Alkoholen; Ketonen; Acetaten; Butyl-Cellosolve; Dimethiconen; Polydimethylsiloxanen; Alkanen; Alkanoaten, dermatologisch verträglichen hydrophoben Esterölen; Dicaprylylcarbonat; Alkylbenzoaten; Hemisqualan; Dioctylether; Ketosäuren und Gemischen davon.

3. Sonnenschutzformulierung nach Anspruch 2, wobei das UV-Strahlung absorbierende Mittel ausgewählt ist aus der Gruppe bestehend aus physikalischen Blockern, chemischen Absorbenzien und Gemischen davon.

4. Sonnenschutzformulierung nach Anspruch 2, wobei das UV-Strahlung absorbierende Mittel ausgewählt ist aus der Gruppe bestehend aus rotem Petrolatum; Titandioxid; Zinkoxid; 1-(4-Methoxyphenol)-3-(4-tert-butylphenyl)propan-1,3-dion; 2-Hydroxy-4-methoxybenzophenon; Dioxybenzon; Sulisobenzon; Menthylanthranilat; para-Aminobenzoesäure; Amylparadimethylaminobenzoesäure; Octyl-para-dimethylaminobenzoat; Ethyl-4-bis(hydroxypropyl)para-aminobenzoat; Polyethylenglycol(PEG-25)para-aminobenzoat; Ethyl-4-bis(hydroxypropyl)aminobenzoat; Diethanolamin-parametyoxycinnamat; 2-Ethoxyethyl-para-methoxycinnamat; Ethylhexylparamethoxycinnamat; Octyl-para-methoxycinnamat; Isoamyl-para-methoxycinnamat; 2-Ethylhexyl-2-cyano-3,3-diphenylacrylat; 2-Ethylhexyl-2-cyano-3,3-diphenyl-2-propenoat; 2-Ethylhexyl-2-hydroxybenzoat; Homomenthylsalicylat; Glycerylaminobenzoat; Triethanolaminsalicylat; Digalloyltrioleat; Lawson mit Dihydroxyaceton; 2-Phenylbenzimidazol-5-sulfonsäure; 4-Methylbenzylidincampher; Avobenzon; Triazinen; Benzotriazolen; vinylgruppenhaltigen Amiden; Zimtsäureamiden; sulfonierten Benzimidazolen); 3,3,5-Trimethylcyclohexyl-2-hydroxybenzoat und Gemischen davon.

5. Sonnenschutzformulierung nach Anspruch 4, wobei das UV-Strahlung absorbierende Mittel ein Gemisch aus UV-Strahlung absorbierenden Mitteln ist, das 1-(4-Methoxyphenol)3-(4-tert-butylphenyl)propan-1,3-dion; 2-Ethylhexyl-2-hydroxybenzoat; 2-Ethylhexyl-2-cyano-3,3-diphenyl-2-propenoat und 3,3,5-Trimethylcyclohexyl-2-hydroxybenzoat einschließt.

6. Sonnenschutzformulierung nach Anspruch 5, wobei der dermatologisch verträgliche Träger einen gerad- oder verzweigtkettigen C₁₋₄-Alkohol einschließt.

7. Sonnenschutzformulierung nach Anspruch 6, wobei der dermatologisch verträgliche Träger Ethanol einschließt.

8. Sonnenschutzformulierung nach Anspruch 7, ferner umfassend ein dermatologisch verträgliches hydrophobes Esteröl.

9. Sonnenschutzformulierung nach Anspruch 8, ferner umfassend einen Farbbestandteil.

## Revendications

1. Formulation de protection solaire, comprenant :
un support acceptable sur le plan dermatologique ;
un agent d'absorption de rayonnement UV ; et
un polymère filmogène, dans laquelle le polymère filmogène est une maltodextrine fonctionnalisée comprenant un polymère de base de maltodextrine fonctionnalisé par des groupes -Si(R¹)₃ ; dans laquelle chaque R¹ est indépendamment un groupe alkyle saturé en C₁₋₁₀, linéaire ou ramifié ; dans laquelle le polymère de base de maltodextrine a un équivalent de dextrose, DE, tel que mesuré par la méthode de Lane et Eynon décrite dans Standard Analytical Method E-26, Corn Refiners Association, 6^{e} édition, 1977, E-26, pages 1-3, de 2 à 20 ; dans laquelle la maltodextrine fonctionnalisée a un degré de substitution de groupes -Si(R¹)₃, DS, de 1,7 à 3 ; et dans laquelle la maltodextrine fonctionnalisée est exempte de carbone vinylique.

2. Formulation de protection solaire selon la revendication 1, dans laquelle le support acceptable sur le plan dermatologique est choisi dans le groupe constitué d'eau ; glycols ; alcools en C₁₋₁₀ à chaîne droite ou ramifiée ; cétones ; acétates ; butyl cellusolve ; diméthicones ; polydiméthylsiloxanes ; alcanes ; alcanoates, huiles d'esters hydrophobes acceptables sur le plan dermatologique ; carbonate de dicaprylyle ; benzoates d'alkyle ; hémisqualane ; dioctyléther ; cétoacides et mélanges de ceux-ci.

3. Formulation de protection solaire selon la revendication 2, dans laquelle l'agent d'absorption de rayonnement UV est choisi dans le groupe constitué de bloqueurs physiques, absorbeurs chimiques et mélanges de ceux-ci.

4. Formulation de protection solaire selon la revendication 2, dans laquelle l'agent d'absorption de rayonnement UV est choisi dans le groupe constitué de vaseline rouge ; dioxyde de titane ; oxyde de zinc ; 1-(4-méthoxyphénol)-3-(4-tert-butylphényl)propane-1,3-dione ; 2-hydroxy-4-méthoxybenzophénone ; dioxybenzone ; sulisobenzone ; anthranilate de menthyle ; acide para-aminobenzoïque ; acide amyl-paradiméthylaminobenzoïque ; para-diméthylaminobenzoate d'octyle ; 4-bis(hydroxypropyl) para-aminobenzoate d'éthyle ; para-aminobenzoate de polyéthylène glycol (PEG-25) ; 4-bis(hydroxypropyl) aminobenzoate d'éthyle ; para-méthoxycinnamate de diéthanolamine ; para-méthoxycinnamate de 2-éthoxyéthyle ; para-méthoxycinnamate d'éthylhexyle ; para-méthoxycinnamate d'octyle ; para-méthoxycinnamate d'isoamyle ; 2-cyano-3,3-diphényl-acrylate de 2-éthylhexyle ; 2-cyano-3,3-diphényl-2-propénoate de 2-éthylhexyle ; 2-hydroxybenzoate de 2-éthylhexyle ; salicylate d'homomenthyle ; aminobenzoate de glycéryle ; salicylate de triéthanolamine ; trioléate de digalloyle ; lawsone avec dihydroxyacétone ; acide 2-phénylbenzimidazole-5-sulfonique ; 4-méthylbenzylidine camphre ; avobenzone ; triazines ; benzotriazoles ; amides à teneur en groupe vinyle ; amides d'acide cinnamique ; benzimidazoles sulfonés) ; 2-hydroxybenzoate de 3,3,5-triméthylcyclohexyle et mélanges de ceux-ci.

5. Formulation de protection solaire selon la revendication 4, dans laquelle l'agent d'absorption de rayonnement UV est un mélange d'agents d'absorption d'UV comportant de la 1-(4-méthoxyphénol)-3-(4-tert-butylphényl)propane-1,3-dione ; du 2-hydroxybenzoate de 2-éthylhexyle ; du 2-cyano-3,3-diphényl-2-propénoate de 2-éthyhexyle et du 2-hydroxybenzoate de 3,3,5-triméthylcyclohexyle.

6. Formulation de protection solaire selon la revendication 5, dans laquelle le support acceptable sur le plan dermatologique comporte un alcool en C₁₋₄ à chaîne linéaire ou ramifiée.

7. Formulation de protection solaire selon la revendication 6, dans laquelle le support acceptable sur le plan dermatologique comporte de l'éthanol.

8. Formulation de protection solaire selon la revendication 7, comprenant en outre une huile d'ester hydrophobe acceptable sur le plan dermatologique.

9. Formulation de protection solaire selon la revendication 8, comprenant en outre un ingrédient colorant.
